# FASCICULE DE BREVET EUROPEEN

(11) **EP 1 992 369 B1**
(45) Date de publication et mention de la délivrance du brevet: **06.05.2015**
(21) Numéro de dépôt: 08290405.3
(22) Date de dépôt: 28.04.2008
(51) Int. Cl.: A61L 27/34, A61L 31/10, A61F 2/24

(54) **Procédé pour la réalisation d'un objet hémocompatible de configuration complexe et objet ainsi obtenu**
Verfahren zur Herstellung eines blutkompatiblen Objekts komplexer Konfiguration und so erhaltenes Objekt
Method of manufacturing a haemocompatible object with complex configuration and object thus obtained

(30) Priorité: 10.05.2007 FR 0703339
(43) Date de publication de la demande: 19.11.2008
(73) Titulaire: CARMAT, 78140 Vélizy Villacoublay (FR)
(72) Inventeur: Capel, Antoine, 92140 Clamart (FR); Carpentier, Alain, 75116 Paris (FR); Melot, Marion, 75012 Paris (FR)
(74) Mandataire: Bonnetat, Christian

(56) Documents cités:
- WO-A-96/16601
- US-A- 4 473 423
- US-A- 6 001 302
- US-A1- 2004 210 307
- US-B1- 6 440 164
- DATABASE WPI Week 200338 Thomson Scientific, London, GB; AN 2003-400764 XP002463567 & RU 2 202 990 C2 (MEDINZH RES PRODN ENTERP) 27 avril 2003 (2003-04-27)

## Description

La présente invention concerne les objets hémocompatibles et, notamment, les prothèses implantables et les revêtements hémocompatibles pour de telles prothèses.

On sait que les surfaces des dispositifs médicaux implantés, en contact direct avec le sang, ne doivent en aucun cas altérer le tissu sanguin, ni perturber le flux du sang. Elles doivent donc être parfaitement hémocompatibles.

On sait, par ailleurs, que les polytétrafluoroéthylènes expansés, généralement désignés dans la technique par e-PTFE, sont largement utilisés pour la réalisation de tels objets hémocompatibles (voir, par exemple, les documents US-4 743 480, WO-95/05277, WO-96/00103, US-5 665 114, EP-O 692 264, US-6 039 755, WO-02/100454 et WO-03/093356). En effet, les e-PTFE présentent des propriétés d'hémocompatibilité remarquables, notamment en ce qui concerne la stabilité chimique et la porosité. Toutefois, leur taux de cristallinité élevé (proche de 95%) et leur structure tridimensionnelle unique constituée de noeuds et de fibres leur confère une mémoire de forme élevée, même à haute température.

Le document WO96/16601 décrit un procédé de fabrication d'une enveloppe de protection d'organe en e-PTFE poreux obtenue par thermoformage.

Le document US4473423 décrit un procédé de fabrication de valve cardiaque par thermoformage.

Aussi, les objets hémocompatibles en e-PTFE que l'on peut obtenir actuellement présentent-ils obligatoirement des formes simples, telles que plaques ou tubes.

La présente invention a pour objet de remédier à cet inconvénient et de permettre d'obtenir des objets hémocompatibles de configuration complexe.

A cette fin, selon l'invention, le procédé pour la réalisation d'un objet hémocompatible, procédé selon lequel :
- on réalise un moule de formage présentant la configuration dudit objet ;
- on conforme une membrane de polytétrafluoroéthylène à la configuration dudit objet en la chauffant et en l'appliquant contre ledit moule de formage au moyen d'une différence de pression engendrée entre les deux faces de ladite membrane ;
- on refroidit ladite membrane ainsi conformée tout en la maintenant appliquée contre ledit moule de formage ; et
- on dégage ladite membrane conformée dudit moule de formage,
est remarquable en ce que :
- ladite membrane est réalisée en un polytétrafluoroéthylène expansé qui n'est pas stabilisé thermiquement, préalablement à sa conformation à la configuration dudit objet, et dont les fibres ne présentent aucune orientation préférentielle ; et
- ladite membrane est chauffée, pendant sa conformation à la configuration dudit objet, jusqu'à une température supérieure au point de gel dudit polytétrafluoroéthylène expansé.

En effet, le demandeur a constaté que, jusqu'à présent, les e-PTFE utilisés pour la réalisation d'objets hémocompatibles ont une structure dans laquelle lesdites fibres présentent une orientation préférentielle et que si, au contraire, on utilise un e-PTFE dont les fibres ne présentent aucune orientation préférentielle, on peut conformer ce e-PTFE par thermoformage, comme cela est usuel pour les polytétrafluoroéthylènes ordinaires (voir, par exemple, WO 96/16601).

Grâce à la présente invention, on peut donc obtenir des objets hémocompatibles en e-PTFE présentant une forme complexe.

Avantageusement, l'échauffement de ladite membrane est réalisé à l'air chaud. La température à laquelle est chauffée ladite membrane peut être de l'ordre de 400°C.

De préférence, l'application de la membrane chauffée contre ledit moule de formage est obtenue par dépression à travers ce dernier.

De plus, ledit refroidissement de la membrane conformée peut être accéléré, par exemple par soufflage d'air froid.

Ledit objet hémocompatible obtenu par la mise en oeuvre de la présente invention peut avantageusement former un revêtement pour une prothèse ou partie de prothèse implantable présentant ladite configuration. Dans ce cas, après dégagement, hors dudit moule de formage, de ladite membrane conformée à la configuration de ladite prothèse ou partie de prothèse, ladite membrane est collée sur cette dernière, par exemple à l'aide d'un élastomère, tel qu'un silicone.

Pour le collage de la membrane conformée sur ladite prothèse ou partie de prothèse, on peut monter ladite membrane conformée sur un outillage gonflable de forme semblable et, après mise en contact de ladite membrane conformée avec ladite prothèse ou partie de prothèse avec interposition d'au moins une couche d'adhésif, ledit outillage est gonflé pour permettre la compression de cette dernière et lui assurer une épaisseur homogène.

Il va de soi que le procédé conforme à la présente invention peut être mis en oeuvre pour la réalisation d'objets hémocompatibles de toutes sortes. Toutefois, dans une application particulièrement intéressante, l'objet hémocompatible constitue un revêtement pour une prothèse cardiaque ou partie de prothèse cardiaque, telle qu'un ventricule artificiel. Ladite prothèse cardiaque est alors remarquable en ce qu'au moins l'une de ses parties comporte un tel revêtement hémocompatible.

Les figures du dessin annexé feront bien comprendre comment l'invention peut être réalisée. Sur ces figures, des références identiques désignent des éléments semblables.

La figure 1 est une représentation schématique, fortement agrandie, de la structure d'un e-PTFE sans orientation préférentielle utilisé dans la présente invention, telle que vue au microscope électronique.

La figure 2 est une coupe schématique d'une partie de prothèse implantable, par exemple un ventricule artificiel pour prothèse cardiaque avec un conduit d'admission et un conduit d'éjection, l'ensemble comportant un revêtement hémocompatible en e-PTFE conforme à la présente invention.

La figure 3 montre en coupe schématique un moule de formage pour obtenir ledit revêtement hémocompatible.

La figure 4 est une vue en perspective dudit moule de formage.

Les figures 5 et 6 illustrent schématiquement le thermoformage d'une membrane réalisée dans le e-PTFE de la figure 1, pour obtenir ledit revêtement hémocompatible.

La figure 7 montre en coupe schématique le revêtement hémocompatible obtenu.

Les figures 8 et 9 illustrent schématiquement la solidarisation dudit revêtement hémocompatible sur ledit ventricule artificiel.

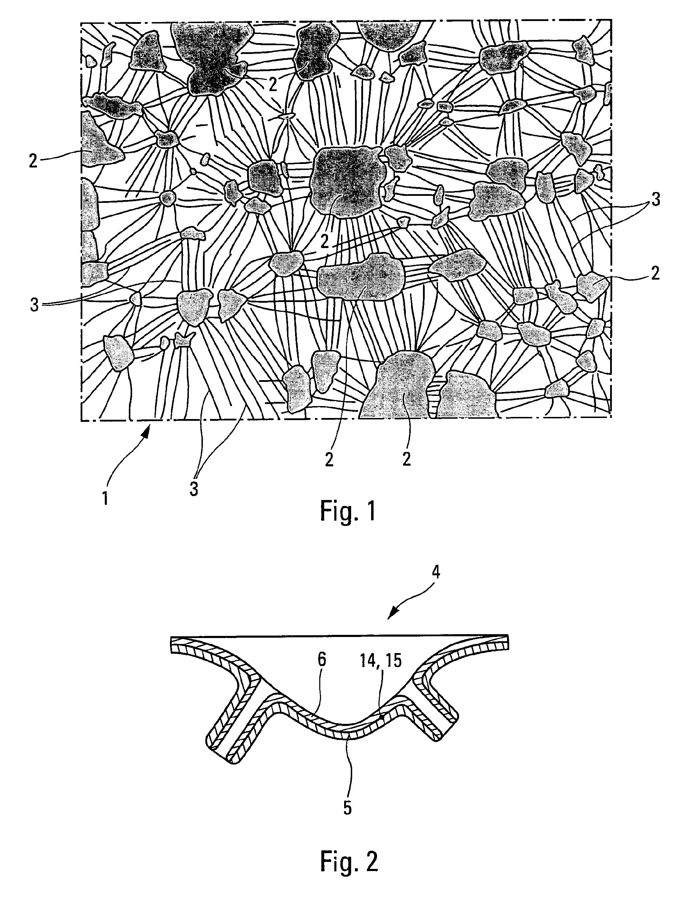

Sur la figure 1, on a représenté schématiquement la vue au microscope électronique d'une partie de la surface d'une membrane 1 en un e-PTFE utilisable dans le procédé de l'invention. Comme on peut le voir, la structure de la membrane 1 comporte une pluralité de noeuds 2 distribués de façon aléatoire et reliés par des fibres 3 multidirectionnelles. Dans la membrane 1 utilisée par l'invention, ni les noeuds 2, ni les fibres 3 ne forment des agencements à orientation préférentielle.

Un e-PTFE, tel que celui dont la structure est illustrée schématiquement sur la figure 1, peut être obtenu, par exemple auprès des sociétés GORE (Gore Medical Product Division, US), TERUMO (Vascutek, Japon) ou BARD (Impra, US).

Un exemple d'application de la présente invention est représenté sur la figure 2, qui montre schématiquement une ébauche de ventricule artificiel 4 pour prothèse cardiaque, comportant une coque rigide 5, par exemple en un métal ou en un matériau plastique tel que le polyétherétherkétone, etc ..., portant un revêtement hémocompatible 6 qui en est solidaire.

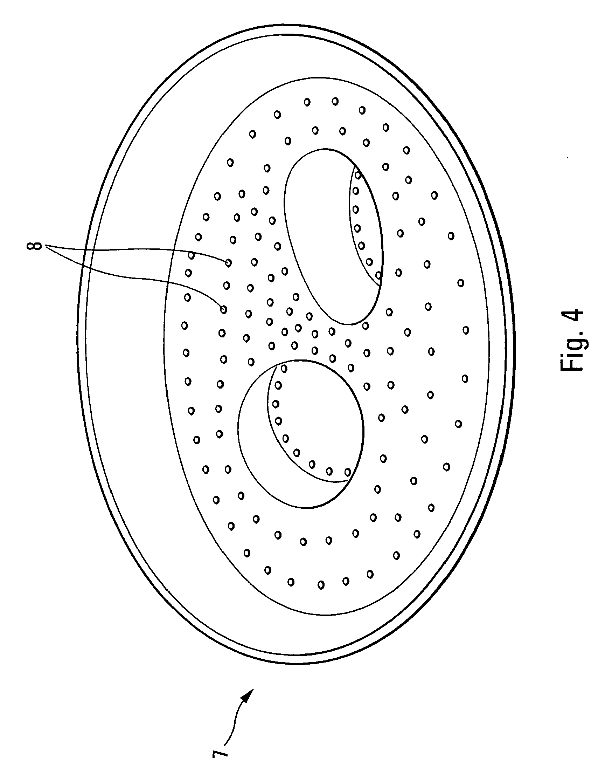

Le revêtement hémocompatible 6 est réalisé à partir de la membrane 1, de la façon illustrée par les figures 3 à 7.

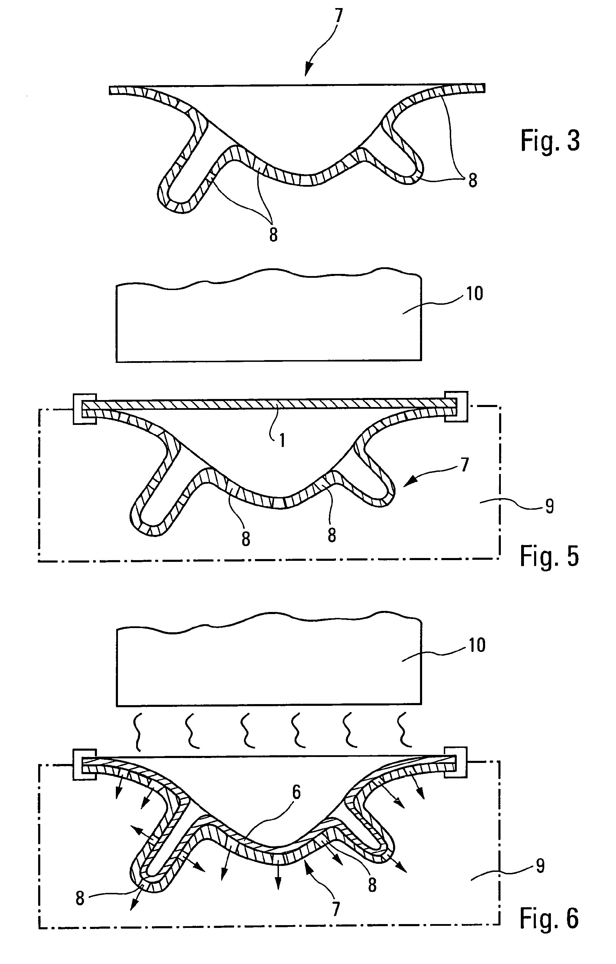

Pour la réalisation du revêtement 6, on utilise un moule de formage, tel que par exemple celui représenté sur les figures 3 et 4 où il porte la référence 7. Le moule de formage 7 est réalisé en un matériau biocompatible et thermocompatible dont la forme reproduit exactement la forme souhaitée pour le revêtement hémocompatible 6. Il est de plus percé par une pluralité d'évents 8.

Comme l'illustre la figure 5, la membrane 1 est fixée de façon étanche sur les bords du moule de formage 7 pour obturer celui-ci, qui est relié à une source de dépression 9. Par ailleurs, un dispositif de chauffage 10 est disposé en regard de la membrane 1.

Ainsi, la membrane 1 peut être chauffée à une température supérieure au point de gel de l'e-PTFE qui la constitue (de l'ordre de 400°C), et plaquée contre le moule de formage 7 par la dépression engendrée par la source 9 à travers les évents 8 (voir la figure 6).

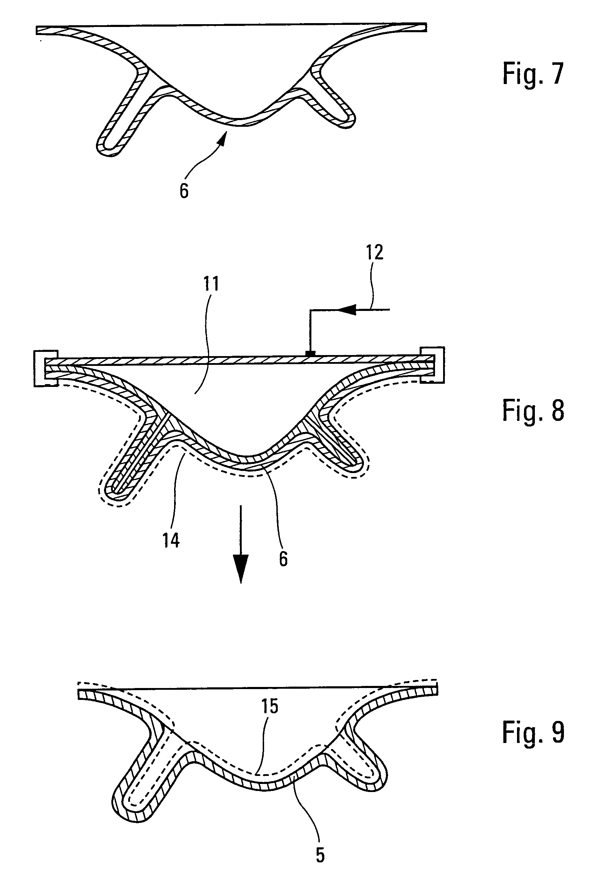

Après conformation de la membrane 1 à la forme du revêtement 6, le chauffage est arrêté, mais la dépression est maintenue pendant le refroidissement de ladite membrane conformée. Eventuellement, le refroidissement de ladite membrane conformée, maintenue plaquée contre ledit moule de formage 7 par la dépression, est accéléré, par exemple par soufflage d'air froid. Lorsque la température ambiante est atteinte, la membrane 1 conformée en revêtement 6 est thermiquement stable et peut être dégagée du moule de formage 7 (voir la figure 7).

En vue de l'adhésion du revêtement 6 sur la coque rigide 5, on monte ledit revêtement 6 sur un outillage gonflable 11, par exemple du type vessie, présentant une configuration identique à cette de ladite coque rigide 5 (voir la figure 8) et pouvant être gonflé grâce à des moyens 12.

Ensuite, les surfaces à coller du revêtement 6 et/ou de la coque rigide 5 sont revêtues d'une couche d'adhésif 14 ou 15, respectivement, par exemple à base de silicone (voir également la figure 9). Préalablement au dépôt desdites couches 14 et 15, lesdites surfaces à solidariser peuvent être traitées pour améliorer ledit collage. Par exemple, à cet effet, ledit revêtement 6 peut être imprégné par dilution d'un élastomère de basse viscosité, par exemple à base de silicone. Dans ce dernier cas, le solvant de ladite dilution est avantageusement éliminé lentement par extraction à chaud et/ou sous vide.

L'ensemble 6, 11, 14 est alors introduit dans la coque 5 pour mettre les couches 14, 15 au contact l'une de l'autre.

L'outillage 11 est alors gonflé pour permettre la compression des couches 14, 15 et assurer une épaisseur homogène au joint d'adhésif qui en résulte.

Après durcissement dudit joint et ouverture des extrémités des conduits d'admission et d'éjection, on obtient l'ébauche de ventricule artificiel de la figure 2.

## Revendications

1. Procédé pour la réalisation d'un objet hémocompatible (6), procédé selon lequel :
- on réalise un moule de formage (7) présentant la configuration dudit objet (6) ;
- on conforme une membrane (1) de polytétrafluoroéthylène à la configuration dudit objet (6) en la chauffant et en l'appliquant contre ledit moule de formage (7) au moyen d'une différence de pression engendrée entre les deux faces de ladite membrane (1) ;
- on refroidit ladite membrane ainsi conformée tout en la maintenant appliquée contre ledit moule de formage (7) ; et
- on dégage ladite membrane conformée dudit moule de formage (7),
**caractérisé en ce que** :
- ladite membrane (1) est réalisée en un polytétrafluoroéthylène expansé qui n'est pas stabilisé thermiquement, préalablement à sa conformation à la configuration dudit objet (6), et dont les fibres (3) ne présentent aucune orientation préférentielle ; et
- ladite membrane (1) est chauffée, pendant sa conformation à la configuration dudit objet (6), jusqu'à une température supérieure au point de gel dudit polytétrafluoroéthylène expansé.

2. Procédé selon la revendication 1,
**caractérisé en ce que** l'échauffement de ladite membrane est réalisé à l'air chaud.

3. Procédé selon l'une des revendications 1 ou 2,
**caractérisé en ce que** la température à laquelle est chauffée ladite membrane (1) est de l'ordre de 400°C.

4. Procédé selon l'une des revendications 1 à 3,
**caractérisé en ce que** ladite application de la membrane chauffée (1) contre ledit moule de formage (7) est obtenue par dépression à travers ce dernier.

5. Procédé selon l'une des revendications 1 à 4,
**caractérisé en ce que** le refroidissement de ladite membrane conformée est accéléré.

6. Procédé selon l'une des revendications précédentes, mis en oeuvre pour la réalisation d'un objet hémocompatible de configuration complexe devant former un revêtement (6) pour une prothèse ou partie de prothèse implantable (4) présentant ladite configuration,
**caractérisé en ce que**, après dégagement, hors dudit moule de formage (7), de ladite membrane conformée à la configuration de ladite prothèse ou partie de prothèse, ladite membrane est collée sur cette dernière.

7. Procédé selon la revendication 6,
**caractérisé en ce que**, pour le collage de la membrane conformée sur ladite prothèse ou partie de prothèse (4), on monte ladite membrane conformée sur un outillage gonflable (11) de forme semblable et, après mise en contact de ladite membrane conformée avec ladite prothèse ou partie de prothèse par l'intermédiaire d'au moins une couche d'adhésif, ledit outillage (11) est gonflé pour permettre la compression de cette dernière et lui assurer une épaisseur homogène.

8. Procédé selon l'une des revendications 6 ou 7,
**caractérisé en ce que** ledit collage est obtenu à l'aide d'un élastomère, tel qu'un silicone.

9. Procédé selon l'une des revendications 1 à 8,
**caractérisé en ce que** ledit objet réalisé est un revêtement hémocompatible (6) recouvrant au moins une des parties d'une prothèse cardiaque.

## Patentansprüche

1. Verfahren zum Herstellen eines hämokompatiblen Objekts (6), Verfahren, gemäß welchem:
- man eine Formgebungsform (7) herstellt, die die Konfiguration des Objekts (6) darstellt,
- man eine Membran (1) aus Polytetrafluorethylen an der Konfiguration des Gegenstands (6) gestaltet , indem man es erhitzt und gegen die Formgebungsform (7) mittels eines Druckunterschieds anlegt, der zwischen den zwei Seiten der Membran (1) erzeugt wird,
- man die so gestaltete Membran abkühlt und gleichzeitig gegen die Formgebungsform (7) angelegt hält, und
- man die gestaltete Membran von der Formgebungsform (7) freigibt,
**dadurch gekennzeichnet, dass**:
- die Membran (1) aus einem expandierten Polytetrafluorethylen hergestellt ist, das vor seiner Formgebung an der Konfiguration des Objekts (6) thermisch nicht stabilisiert ist und dessen Fasern (3) keine bevorzugte Ausrichtung aufweisen, und
- die Membran (1) während ihrer Formgebung an der Konfiguration des Gegenstands (6) bis auf eine Temperatur, die höher ist als der Gelierpunkt des expandierten Polytetrafluorethylens erhitzt wird.

2. Verfahren nach Anspruch 1,
**dadurch gekennzeichnet, dass** das Erhitzen der Membran mit Heißluft erfolgt.

3. Verfahren nach einem der Ansprüche 1 oder 2,
**dadurch gekennzeichnet, dass** die Temperatur, auf die die Membran (1) erhitzt wird, in der Größenordnung von 400 °C liegt.

4. Verfahren nach einem der Ansprüche 1 bis 3,
**dadurch gekennzeichnet, dass** das Anlegen der erhitzten Membran (1) gegen die Formgebungsform (7) durch Unterdruck durch diese Letztere hindurch erzielt wird.

5. Verfahren nach einem der Ansprüche 1 bis 4,
**dadurch gekennzeichnet, dass** das Abkühlen der gestalteten Membran beschleunigt ist.

6. Verfahren nach einem der vorhergehenden Ansprüche, das für die Herstellung eines hämokompatiblen Objekts mit komplexer Konfiguration umgesetzt wird, das eine Beschichtung (6) für eine Prothese oder einen Teil einer implantierbaren Prothese (4), die die Konfiguration aufweist, bilden soll,
**dadurch gekennzeichnet, dass** die auf die Konfiguration der Prothese oder des Prothesenteils gestaltete Membran nach dem Freigeben aus der Formgebungsform (7) auf diese Letztere geklebt wird.

7. Verfahren nach Anspruch 6, **dadurch gekennzeichnet, dass** man für das Kleben der auf der Prothese oder dem Prothesenteil (4) gestalteten Membran die gestaltete Membran auf ein aufblasbares Werkzeug (11) mit ähnlicher Form montiert und, nach Inberührungbringen der gestalteten Membran mit der Prothese oder dem Prothesenteil über mindestens eine Klebstoffschicht, das Werkzeug (11) aufgeblasen wird, um das Komprimieren dieser Letzteren zu erlauben und ihr eine homogene Stärke sicherzustellen.

8. Verfahren nach einem der Ansprüche 6 oder 7,
**dadurch gekennzeichnet, dass** das Kleben mit Hilfe eines Elastomers wie zum Beispiel eines Silikons erzielt wird.

9. Verfahren nach einem der Ansprüche 1 bis 8,
**dadurch gekennzeichnet, dass** das hergestellte Objekt eine hämokompatible Beschichtung (6) ist, die mindestens einen der Teile einer Herzprothese abdeckt.

## Claims

1. A method for producing a hemocompatible article (6), in which method:
- a forming mold (7) having the configuration of said article (6) is produced;
- a polytetrafluoroethylene membrane (1) is conformed to the configuration of said article (6) by heating it and applying it against said forming mold (7) by means of a pressure difference generated between the two faces of said membrane (1);
- said membrane thus conformed is cooled while still keeping it applied against said forming mold (7); and
- said conformed membrane is removed from said forming mold (7),
**characterized in that**:
- said membrane (1) is made of an expanded polytetrafluoroethylene which has not been thermally stabilized, prior to its conformation to the configuration of said article (6), and the fibers (3) of which membrane have no preferred orientation; and
- said membrane (1) is heated while it is being conformed to the configuration of said article (6), up to a temperature above the gel point of said expanded polytetrafluoroethylene.

2. The method as claimed in claim 1, **characterized in that** said membrane is heated with hot air.

3. The method as claimed in either of claims 1 and 2, **characterized in that** the temperature to which said membrane (1) is heated is around 400°C.

4. The method as claimed in one of claims 1 to 3, **characterized in that** said heated membrane (1) is applied against said forming mold (7) by a vacuum through the latter.

5. The method as claimed in one of claims 1 to 4, **characterized in that** said cooling of the conformed membrane is accelerated cooling.

6. The method as claimed in one of the preceding claims, implemented for producing a hemocompatible article of complex configuration that has to form a coating (6) for an implantable prosthesis or prosthesis part (4) having said configuration, **characterized in that**, after said membrane conformed to the configuration of said prosthesis or prosthesis part has been removed from said forming mold (7), said membrane is bonded to said prosthesis or prosthesis part.

7. The method as claimed in claim 6, **characterized in that**, for bonding the conformed membrane to said prosthesis or prosthesis part (4), said conformed membrane is mounted on an inflatable tool (11) of similar shape and, after said conformed membrane has been brought into contact with said prosthesis or prosthesis part via at least one layer of adhesive, said tool (11) is inflated so as to compress said layer and ensure that it has a uniform thickness.

8. The method as claimed in either of claims 6 and 7, **characterized in that** said bonding is obtained by means of an elastomer, such as a silicone.

9. The method as claimed in one of claims 1 to 8, **characterized in that** said produced article is a hemocompatible coating (6) covering at least one part of a cardiac prosthesis.
